# EUROPEAN PATENT SPECIFICATION

(11) **EP 0 688 869 B1**
(45) Date of publication and mention of the grant of the patent: **19.03.2003**
(21) Application number: 95111771.2
(22) Date of filing: 30.06.1987
(51) Int. Cl.: C12N 15/12, C07K 14/51, C12N 5/10, C12N 1/21, A61K 38/18

(54) **Novel osteoinductive compositions**
Osteoinduktive Zusammensetzungen
Compositions ostéoinductrices

(30) Priority: 01.07.1986 US 880776; 17.12.1986 US 943332; 20.03.1987 US 28285; 26.03.1987 US 31346
(43) Date of publication of application: 27.12.1995
(62) Divisional of application: 87905023.5
(73) Proprietor: Genetics Institute, LLC, Cambridge, MA 02140 (US)
(72) Inventor: Wang, Elizabeth A., Carlisle, MA 01741 (US); Wozney, John M., Hudson, MA 01749 (US); Rosen, Vicki A., Boston, MA 02116 (US)
(74) Representative: VOSSIUS & PARTNER

(56) References cited:
- US-A- 4 455 256

## Description

The present invention relates to novel proteins and processes for obtaining them. These proteins are capable of inducing cartilage and bone formation.

### Background

Bone is a highly specialized tissue characterized by an extensive matrix structure formed of fibrous bundles of the protein collagen, and proteoglycans, noncollagenous proteins, lipids and acidic proteins. The processes of bone formation and renewal/repair of bone tissue, which occur continuously throughout life, are performed by specialized cells. Normal embryonic long bone development is preceded by formation of a cartilage model. Bone growth is presumably mediated by "osteoblasts" (bone-forming cells), while remodeling of bone is apparently accomplished by the joint activities of bone-resorbing cells, called "osteoclasts" and osteoblasts. A variety of osteogenic, cartilage-inducing and bone inducing factors have been described. See, e.g. European patent applications 148,155 and 169,016 for discussions thereof.

### Brief Description of the Invention

The present invention provides a novel protein in purified form BMP-3, wherein BMP is bone morphogenic protein. This protein is characterized by peptide sequences the same as or substantially homologous to amino acid sequences illustrated in Tables IV A+B below. It is capable of inducing bone formation at a predetermined site. This bone inductive factor is further characterized by biochemical and biological characteristics including activity at a concentration of 10 to 1000ng/gram of bone in an in vivo rat bone formation assay described below. Proteins of this invention may be encoded by the DNA sequences depicted in the Tables or by sequences capable of hybridizing thereto unden strigent conditions and coding for polypeptides with bone growth factor biological properties or other variously modified sequences demonstrating such properties.

The pone inductive factor of the invention, BMP-3, is represented by the bovine homolog bBMP-3. bBMP-3 is characterized by the DNA sequence and amino acid sequence of Table IV A and B which represents the bovine genomic sequence. It is characterized by at least a portion of a peptide sequence the same or substantially the same as amino acid #1 through amino acid #175 of Table IV A and B. BMP-3 is further characterized by the ability to induce bone formation. The bovine factor may be employed as a tool for obtaining the analogous human BMP-3 protein or other mammalian bone inductive proteins. The proper characterization of this bovine bone inductive factor provides the essential "starting point" for the method employing this sequence. The method, employing techniques known to those skilled in the art of genetic engineering, involves using the bovine DNA sequence as a probe to screen a human genomic or cDNA library; and identifying the DNA sequences which hybridize to the probes. A clone with a hybridizable sequence is plaque purified and the DNA isolated therefrom, subcloned and subjected to DNA sequence analysis. Thus another aspect of this invention is a human protein hBMP-3, produced by this method.

Another aspect of the invention provides pharmaceutical compositions containing a therapeutically effective amount of the bone growth factor polypeptide according to the invention in a pharmaceutically acceptable vehicle.. These compositions may further include other therapeutically useful agents. They may also include an appropriate matrix for delivering the proteins to the site of the bone defect and for providing a structure for'bone growth. These compositions may be employed in methods for treating a number of bone defects and periodontal disease. These methods, according to the invention, entail administering to a patient needing such bone formation an effective amount of the novel protein BMP-3 as described herein.

Still a further aspect of the invention are DNA sequences coding on expression for a human or bovine polypeptide having the ability to induce bone formation. Such sequences include the sequence or nucleotides in a 5' to 3' direction illustrated in Tables IV A+B. Alternatively, a DNA sequence which hybridizes under stringent conditions with the DNA sequences of Tables IV A+B and which codes on expression for a protein having at least one bone growth factor biological property are included in the present invention. Finally, allelic or other variations of the sequences of Tables IV A+B, whether such nucleotide changes result in changes in the peptide sequence or not, are also included in the present invention.

Still a further aspect of the invention is a vector containing a DNA sequence as described above in operative association with an expression control sequence. Such vector may be employed in a novel process for producing a bone growth factor polypeptide in which a cell line transformed with a DNA sequence encoding expression of a bone growth factor polypeptide in operative association with an expression control sequence therefor, is cultured. This claimed process may employ a number of known cells as host cells for expression of the polypeptide. Presently preferred cell lines are mammalian cell lines and bacterial cells.

Other aspects and advantages of the present invention will be apparent upon consideration of the following detailed description and preferred embodiments thereof.

### Detailed Description of the Invention

The protein of the present invention is characterized by amino acid sequences or portions thereof the same as or substantially homologous to the sequences shown in Tables IV A+B below. This protein is also characterized by the ability to induce bone formation.

The bone growth factors provided herein also include factors encoded by the sequences similar to those of Tables IV A+B, but into which modifications are naturally provided (e.g. allelic variations in the nucleotide sequence which may result in amino acid changes in the polypeptide) or deliberately engineered. For example, synthetic polypeptides may wholly or partially duplicate continuous sequences of the amino acid residues of Tables IV A+B. These sequences, by virtue of sharing primary, secondary, or tertiary structural and conformational characteristics with the bone growth factor polypeptide of Tables IV A+B may possess bone growth factor biological properties in common therewith. Thus, they may be employed as biologically active substitutes for naturally-occurring bone growth factor polypeptides in therapeutic processes.

Other specific mutations of the sequences of the bone growth factor described herein involve modifications of one or both of the glycosylation sites. The absence of glycosylation or only partial glycosylation results from amino acid substitution or deletion at one or both of the asparagine-linked glycosylation recognition sites present in the sequences of the bone growth factor shown in Tables IV A+B. The asparagine-linked glycosylation recognition sites comprise tripeptide sequences which are specifically recognized by appropriate cellular glycosylation enzymes. These tripeptide sequences are either asparagine-X-threonine or asparagine-X-serine, where X is usually any amino acid. A variety of amino acid substitutions or deletions at one or both of the first or third amino acid positions of a glycosylation recognition site (and/or amino acid deletion at the second position) results in non-glycosylation at the modified tripeptide sequence.

The present invention also encompasses the novel DNA sequences, free of association with DNA sequences encoding other proteinaceous materials, and coding on expression for bone growth factors. These DNA sequences include those depicted in Tables IV A+B in a 5' to 3' direction and those sequences which hybridize under stringent hybridization conditions [see, T. Maniatis et al, Molecular Cloning (A Laboratory Manual), Cold Spring Harbor Laboratory (1982), pages 387 to 389] to the DNA sequences of Tables IV A+B.

Similarly, DNA sequences which code for bone growth factor polypeptides coded for by the sequences of Tables IV A+B, but which differ in codon sequence due to the degeneracies of the genetic code or allelic variations (naturally-occurring base changes in the species population which may or may not result in an amino acid change) also encode the novel growth factors described herein. Variations in the DNA sequences of Tables IV A+B which are caused by point mutations or by induced modifications to enhance the activity, half-life or production of the polypeptides encoded thereby are also encompassed in the invention.

Another aspect of the present invention provides a novel method for producing the novel osteoinductive factors. The method of the present invention involves culturing a suitable cell or cell line, which has been transformed with a DNA sequence coding on expression for a novel bone growth factor polypeptide of the invention, under the control of known regulatory sequences. Suitable cells or cell lines may be mammalian cells, such as Chinese hamster ovary cells (CHO). The selection of suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U.S. Patent 4,419,446. Another suitable mammalian cell line, which is described in the accompanying examples, is the monkey COS-1 cell line. A similarly useful mammalian cell line is the CV-1 cell line.

Bacterial cells are suitable hosts. For example, the various strains of E. coli (e.g., HB101, MC1061) are well-known as host cells in the field of biotechnology. Various strains of B. subtilis, Pseudomonas, other bacilli and the like may also be employed in this method.

Many strains of yeast cells known to those skilled in the art are also available as host cells for expression of the polypeptides of the present invention. Additionally, where desired, insect cells may be utilized as host cells in the method of the present invention. See, e.g. Miller et al, Genetic Engineering, 8:277-298 (Plenum Press 1986) and references, cited therein.

Another aspect of the present invention provides vectors for use in the method of expression of these novel osteoinductive polypeptides. Preferably the vectors contain the full novel DNA sequences described above which code for the novel factors of the invention. Additionally the vectors also contain appropriate expression control sequences permitting expression of the bone inductive protein sequences. Alternatively, vectors incorporating modified sequences as described above are also embodiments of the present invention and useful in the production of the bone inductive proteins. The vectors may be employed in the method of transforming cell lines and contain selected regulatory sequences in operative association with the DNA coding sequences of the invention which are capable of directing the replication and expression thereof in selected host cells. Useful regulatory sequences for such vectors are known to one of skill in the art and may be selected depending upon the selected host cells. Such selection is routine and does not form part of the present invention.

A protein of the present invention, which induces bone growth in circumstances where bone is not normally formed, has application'in the healing of bone fractures. An osteogenic preparation employing is protein of the invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery. An osteogenic factor of the invention may be valuable in the treatment of periodontal disease, and in other tooth repair processes. Such agents may provide an environment to attract bone-forming cells, stimulate growth of bone-forming cells or induce differentiation of progenitors of bone-forming cells. Of course, the proteins of the invention may have other therapeutic uses.

A further aspect of the invention is a therapeutic method and composition for repairing fractures and other conditions related to bone defects or periodontal diseases. Such a composition comprises a therapeutically effective amount of the bone inductive factor protein of the invention. The bone inductive factor according to the present invention may be present in a therapeutic composition in admixture with a pharmaceutically acceptable vehicle or matrix. Further therapeutic methods and compositions of the invention comprise a therapeutic amount of a bone inductive factor of the invention with a therapeutic amount of at least one of the other bone inductive factors of the invention. Additionally, the protein according to the present invention may be co-administered with one or more different osteoinductive factors with which it may interact. Further, the bone inductive protein may be combined with other agents beneficial to the treatment of the bone defect in question. Such agents include, but are not limited to various growth factors. The preparation of such physiologically acceptable protein compositions, having due regard to pH, isotonicity, stability and the like, is within the skill of the art.

The therapeutic method includes locally administering the composition as an implant or device. When administered, the therapeutic composition for use in this invention is, of course, in a pyrogen-free, physiologically acceptable form. Further, the composition may desirably be encapsulated or injected in a viscous form for delivery to the site of bone damage. Preferably, the bone growth inductive factor composition would include a matrix capable of delivering the bone inductive factor to the site of bone damage, providing a structure for the developing bone and cartilage and optimally capable of being resorbed into the body. Such matrices may be formed of other materials presently in use for other implanted medical applications.

The choice of material is based on, for example, biocompatibility, biodegradability, mechanical properties, cosmetic appearance and interface properties. Similarly, the application of the osteoinductive factors will define the appropriate formulation. Potential matrices for the osteoinductive factors may be biodegradable and chemically defined, such as, but not limited to calcium sulfate, tricalciumphosphate, hydroxyapatite, polylactic acid, polyanhydrides; biodegradable and biologically well defined, such as bone or dermal collagen, other pure proteins or extracellular matrix components; nonbiodegradable and chemically defined, such as sintered hydroxyapatite, bioglass, aluminates, or other ceramics; or combinations of any of the above mentioned types of material, such as polylactic acid and hydroxyapatite or collagen and tricalciumphosphate. The bioceramics might also be altered in composition, such as in calcium-aluminate-phosphate and processing to alter for example, pore size, particle size, particle shape, and biodegradability.

The dosage regimen will be determined by the attending physician considering various factors which modify the action of such a growth factor, e.g. amount of bone weight desired to be formed, the site of bone damage, the condition of the damaged bone, the patient's age, sex, and diet, the severity of any infection, time of administration and other clinical factors. The dosage may vary with the type of matrix used in the reconstitution. The addition of other known growth factors, such as IGF 1 (insulin like growth factor 1), to the final composition, may also effect the dosage. Generally, the dosage regimen should be in the range of approximately 10 to 10⁶ nanograms of protein per gram of bone weight desired. Progress can be monitored by periodic assessment of bone growth and/or repair, e.g. x-rays. Such therapeutic compositions are also presently valuable for veterinary applications due to the lack of species specificity in bone inductive factors. Particularly domestic animals and thoroughbred horses in addition to humans are desired patients for such treatment with the bone inductive factors of the present invention.

The following examples illustrate practice of the present invention in recovering and characterizing the bovine proteins and employing them to recover the human proteins, obtaining the human proteins and in expressing the proteins via recombinant techniques.

### EXAMPLE I

### Isolation of Bovine Bone Inductive Factor

Ground bovine bone powder (20-120 mesh, Helitrex) is prepared according to the procedures of M. R. Urist et al., Proc. Natl Acad. Sci USA, 70:3511 (1973) with elimination of some extraction steps as identified below. Ten kgs of the ground powder is demineralized in successive changes of 0.6N HCl at 4°C over a 48 hour period with vigorous stirring. The resulting suspension is extracted for 16 hours at 4°C with 50 liters of 2M CaCl₂ and 10mM ethylenediamine-tetraacetic acid [EDTA], and followed by extraction for 4 hours in 50 liters of 0.5M EDTA. The residue is washed three times with distilled water before its resuspension in 20 liters of 4M guanidine hydrochloride [GuCl], 20mM Tris (pH 7.4), 1mM N-ethylmaleimide, 1mM iodoacetamide, 1mM phenylmethylsulfonyl fluorine as described in Clin. Orthop. Rel. Res., 171: 213 (1982). After 16 to 20 hours the supernatant is removed and replaced with another 10 liters of GuCl buffer. The residue is extracted for another 24 hours.

The crude GuCl extracts are combined, concentrated approximately 20 times on a Pellicon apparatus with a 10,000 molecular weight cut-off membrane, and then dialyzed in 50mM Tris, 0.1M NaCl, 6M urea (pH7.2), the starting buffer for the first column. After extensive dialysis the protein is loaded on a 4 liter DEAF cellulose column and the unbound fractions are collected.

The unbound fractions are concentrated and dialyzed against 50mM NaAc, 50mM NaCl (pH 4.6) in 6M urea. The unbound fractions are applied to a carboxymethyl cellulose column. Protein not bound to the column is removed by extensive washing with starting buffer, and the bone inductive factor containing material desorbed from the column by 50mM NaAc, 0.25mM NaCl, 6M urea (pH 4.6). The protein from this step elution is concentrated 20- to 40- fold, then diluted 5 times with 80mM KPO₄, 6M urea (pH6.0). The pH of the solution is adjusted to 6.0 with 500mM K₂HPO₄. The sample is applied to an hydroxylapatite column (LKB) equilibrated in 80mM KPO₄, 6M urea (pH6.0) and all unbound protein is removed by washing the column with the same buffer. Bone inductive factor activity is eluted with 100mM KPO₄ (pH7.4) and 6M urea.

The protein is concentrated approximately 10 times, and solid NaCl added to a final concentration of 0.15M. This material is applied to a heparin - Sepharose column equilibrated in 50mM KPO₄, 150mM NaCl, 6M urea (pH7.4). After extensive washing of the column with starting buffer, a protein with bone inductive factor activity is eluted by 50mM KPO₄, 700mM NaCl, 6M urea (pH7.4). This fraction is concentrated to a minimum volume, and 0.4ml aliquots are applied to Superose 6 and Superose 12 columns connected in series, equilibrated with 4M GuCl, 20mM Tris (pH7.2) and the columns developed at a flow rate of 0.25ml/min. The protein demonstrating bone inductive factor activity has a relative migration corresponding to approximately 30,000 dalton protein.

The above fractions are pooled, dialyzed against 50mM NaAc, 6M urea (pH4.6), and applied to a Pharmacia MonoS HR column. The column is developed with a gradient to 1.0M NaCl, 50mM NaAc, 6M urea (pH4.6). Active fractions are pooled and brought to pH3.0 with 10% trifluoroacetic acid (TFA). The material is applied to a 0.46 x 25cm Vydac C4 column in 0.1% TFA and the column developed with a gradient to 90% acetonitrile, 0.1% TFA (31.5% acetonitrile, 0.1% TFA to 49.5% acetonitrile, 0.1% TFA in 60 minutes at 1ml per minute). Active material is eluted at approximately 40-44% acetonitrile. Aliquots of the appropriate fractions are iodinated by one of the following methods: P. J. McConahey et al, Int. Arch. Allergy, 29:185-189 (1966); A. E. Bolton et al, Biochem J., 133:529 (1973); and D. F. Bowen-Pope, J. Biol. Chem., 237:5161 (1982). The iodinated proteins present in these fractions are analyzed by SDS gel electrophoresis and urea Triton X 100 isoelectric focusing. At this stage, the bone inductive factor is estimated to be approximately 10-50% pure.

### EXAMPLE II

### Characterization of Bovine Bone Inductive Factor

### A. Molecular Weight

Approximately 20ug protein from Example I is lyophilized and redissolved in 1X SDS sample buffer. After 15 minutes of heating at 37°C, the sample is applied to a 15% SDS polyacrylamide gel and then electrophoresed with cooling. The molecular weight is determined relative to prestained molecular weight standards (Bethesda Research Labs). Immediately after completion, the gel lane containing bone inductive factor is sliced into 0.3cm pieces. Each piece is mashed and 1.4ml of 0.1% SDS is added. The samples are shaken gently overnight at room temperature to elute the protein. Each gel slice is desalted to prevent interference in the biological assay. The supernatant from each sample is acidified to pH 3.0 with 10% TFA, filtered through a 0.45 micron membrane and loaded on a 0.46cm x 5cm C4 Vydac column developed with a gradient of 0.1% TFA to 0.1% TFA, 90% CH₃CN. The appropriate bone inductive factor - containing fractions are pooled and reconstituted with 20mg rat matrix. In this gel system, the majority of bone inductive factor fractions have the mobility of a protein having a molecular weight of approximately 28,000 - 30,000 daltons.

### B. Isoelectric Focusing

The isoelectric point of bone inductive factor activity is determined in a denaturing isoelectric focusing system. The Triton X100 urea gel system (Hoeffer Scientific) is modified as follows: 1) 40% of the ampholytes used are Servalyte 3/10; 60% are Servalyte 7-9. 2) The catholyte used is 40mM NaOH. Approximately 20ug of protein from Example I is lyophilized, dissolved in sample buffer and applied to the isoelectrofocusing gel. The gel is run at 20 watts, 10°C for approximately 3 hours. At completion the lane containing bone inductive factor is sliced into 0.5 cm slices. Each piece is mashed in 1.0ml 6M urea, 5mM Tris (pH 7.8) and the samples agitated at room temperature. The samples are acidified, filtered, desalted and assayed as described above. The major portion of activity as determined in the assay described in Example III migrates in a manner consistent with a pI of 8.8 - 9.2.

### C. Subunit Characterization

The subunit composition of bone inductive factor is also determined. Pure bone inductive factor is isolated from a preparative 15% SDS gel as described above. A portion of the sample is then reduced with 5mM DTT in sample buffer and re-electrophoresed on a 15% SDS gel. The approximately 30kd protein yields two major bands at approximately 20kd and 18kd, as well as a minor band at 30kd. The broadness of the two bands indicates heterogeneity caused most probably by glycosylation, other post translational modification, proteolytic degradation or carbamylation.

### EXAMPLE III

### Biological Activity of Bone Inductive Factor

A rat bone formation assay according to the general procedure of Sampath and Reddi, Proc. Natl. Acad. Sci. U.S.A., 80:6591-6595 (1983) is used to evaluate the osteogenic activity of the bovine bone inductive factor of the present invention obtained in Example I. This assay can also be used to evaluate bone inductive factors of other species. The ethanol precipitation step is replaced by dialyzing the fraction to be assayed against water. The solution or suspension is then redissolved in a volatile solvent, e.g. 0.1 - 0.2 % TFA, and the resulting solution added to 20mg of rat matrix. This material is frozen and lyophilized and the resulting powder enclosed in #5 gelatin capsules. The capsules are implanted subcutaneously in the abdominal thoracic area of 21 - 49 day old male long Evans rats. The implants are removed after 7-14 days. Half of each implant is used for alkaline phosphatase analysis [See, A. H. Reddi et al., Proc. Natl Acad Sci., 69:1601 (1972)] and half is fixed and processed for histological analysis. Routinely, lum glycolmethacrylate sections are stained with Von Kossa and acid fuschin to detect new bone mineral. Alkaline phosphatase, an enzyme produced by chondroblasts and osteoblasts in the process of matrix formation, is also measured. New cartilage and bone formation often correlates with alkaline phosphatase levels. Table I below illustrates the dose response of the rat matrix samples including a control not treated with bone inductive factor.

**TABLE 1**

| Protein∗ Implanted ug | Cartilage | Alk. Phos.u/l |
|---|---|---|
| 7.5 | 2 | Not done |
| 2.5 | 3 | 445.7 |
| 0.83 | 3 | 77.4 |
| 0.28 | 0 | 32.5 |
| 0.00 | 0 | 31.0 |

| | | |
|---|---|---|
| ∗At this stage the bone inductive factor is approximately 10-15% pure. | | |

The bone or cartilage formed is physically confined to the space occupied by the matrix. Samples are also analyzed by SDS gel electrophoresis and isoelectric focusing as described above, followed by autoradiography. Analysis reveals a correlation of activity with protein bands at 28 - 30kd and a pI 9.0. An extinction coefficient of 1 OD/mg-cm is used as an estimate for protein and approximating the purity of bone inductive factor in a particular fraction. In the in vivo rat bone formation assays on dilutions as described above, the protein is active in vivo at 10 to 200ng protein/gram bone to probably greater than lug protein/gram bone.

### EXAMPLE IV

### Bovine Bone Inductive Factor Protein Composition

The protein composition of Example IIA of molecular weight 28 - 30kd is reduced as described in Example IIC and digested with trypsin. Eight tryptic fragments are isolated by standard procedures having the following amino acid sequences:
Fragment 1: A A F L G D I A L D E E D L G
Fragment 2: A F Q V Q Q A A D L
Fragment 3: N Y Q D M V V E G
Fragment 4: S T P A Q D V S R
Fragment 5: N Q E A L R
Fragment 6: L S E P D P S H T L E E
Fragment 7: F D A Y Y
Fragment 8: L K P S N ? A T I Q S I V E

A less highly purified preparation of protein from bovine bone is prepared according to a purification scheme similar to that described in Example I. The purification basically varies from that previously described by omission of the DE-52 column, the CM cellulose column and the mono S column, as well as a reversal in the order of the hydroxylapatite and heparin sepharose columns. Briefly, the concentrted crude 4 M extract is brought to 85% final concentration of ethanol at 4 degrees. The mixture is then centrifuged, and the precipitate redissolved in 50 mM Tris, 0.15 M NaCl, 6.0 M urea. This material is then fractionated on Heparin Sepharose as described. The Heparin bound material is fractionated on hydroxyapatite as described. The active fractions are pooled, concentrated, and fractionated on a high resolution gel filtration (TSK 30000 in 6 M guanidinium chloride, 50 mM Tris, pH 7.2). The active fractions are pooled, dialyzed against 0.1% TFA, and then fractionated on a C4 Vydac reverse phase column as described. The preparation is reduced and electrophoresed on an acrylamide gel. The protein corresponding to the 18K band is eluted and digested with trypsin. Tryptic fragments are isolated having the following amino acid sequences:
Fragment 9: S L K P S N H A T I Q S ? V
Fragment 10: S F D A Y Y C S ? A
Fragment 11: V Y P N M T V E S C A
Fragment 12: V D F A D I ? W

Tryptic Fragments 7 and 8 are noted to be substantially the same as Fragments 10 and 9, respectively.

### A. bBMP-3

Probes consisting of pools of oligonucleotides are designed on the basis of the amino acid sequences of the tryptic Fragments 9 (Probe #3), 10 (Probe #2), and 11 (Probe #1), and synthesized on an automated DNA synthesizer.

Probe #1: A C N G T C A T [A/G] T T N G G [A/G] T A

Probe #2: C A [A/G] T A [A/G] T A N G C [A/G] T C [A/G] A A

Probe #3: T G [A/G/T] A T N G T N G C [A/G] T G [A/G] T T

A bovine genomic recombinant library is constructed as follows: Bovine liver DNA is partially digested with the restriction endonuclease enzyme Sau 3A and sedimented through a sucrose gradient. Size fractionated DNA in the range of 15-30kb is then ligated to the bacteriophage Bam HI vector EMBL3 [Frischauf et al, J. Mol. Biol., 170:827-842 (1983)]. The library is plated at 8000 recombinants per plate. Duplicate nitrocellulose replicas of the plaques are made and amplified according to a modification of the procedure of Woo et al, Proc. Natl. Acad. Sci. USA, 75:3688-91 (1978).

This recombinant bovine genomic library constructed in EMBL3 is screened by the TMAC hybridization procedure, i.e. kyloridized in 3M tetramethylammonium chloride (TMAC), 0.1M sodium phosphate pH6.5, 1mM EDTA, 5X Denhardts, 0.6% SDS, 100ug/ml salmon sperm DNA at 48 degrees C, and washed in 3M TMAC, 50mM Tris pH8.0 at 50 degrees C. These conditions minimize the detection of mismatches to the 17 mer probe pool [see, Wood et al, Proc. Natl. Acad. Sci, U.S.A., 82:1585-1588 (1985)]. 400,000 recombinants are screened in duplicate with Probe #1 which has been labeled with ³²P. All recombinants which hybridized to this probe are replated for secondaries. Triplicate nitrocellulose replicas are made of the secondary plates, and amplified as described. The three sets of filters are hybridized to Probes #1, #2 and #3, again under TMAC conditions. One clone, lambda bP-819, hybridizes to all three probes and is plaque purified and DNA is isolated from a plate lysate. Bacteriophage lambda bP-819 was deposited with the ATCC on June 16, 1987 under accession number 40344. This bP-819 clone encodes the bovine bone growth factor designated bBMP-3.

The region of bP-819 which hybridizes to Probe #2 is localized and. sequenced. The partial DNA and derived amino acid sequences of this region are shown in Table IVA. The amino acid sequences corresponding to tryptic Fragments 10 and 12 are underlined. The first underlined sequence corresponds to Fragment 12 while the second corresponds to Fragment 10. This region of bP-819, therefore, which hybridizes to Probe #2 encodes at least 111 amino acids. This amino acid sequence is encoded by the DNA sequence from nucleotide #414 through #746.

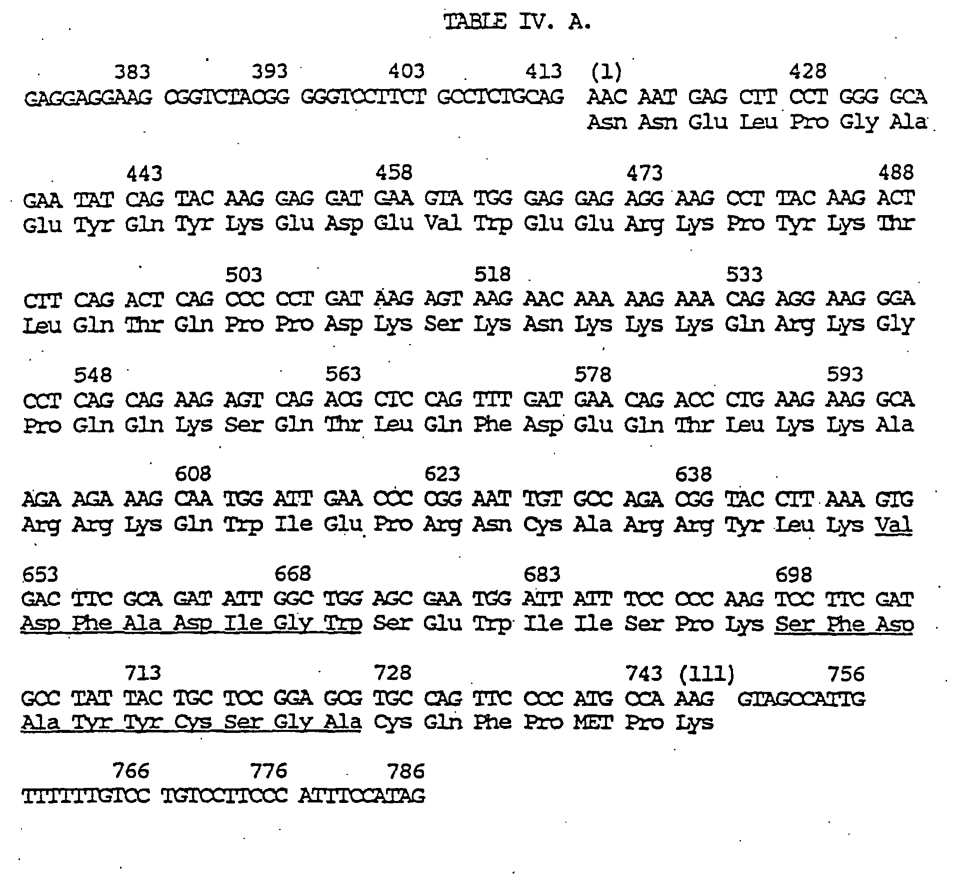

The region of bP-819 which hybridizes to Probe #1 and #3 is localized and sequenced. The partial DNA and derived amino acid sequences of this region are shown in Table IVB. The amino acid sequences corresponding to tryptic Fragments 9 and 11 are underlined. The first underlined sequence corresponds to Fragment 9 while the second underlined sequence corresponds to Fragment 11. The peptide sequence of this region of bP-819 which hybridizes to Probe #1 and #3 is 64 amino acids in length encoded by nucleotide #305 through #493 of Table IVB. The arginine residue encoded by the AGA triplet is presumed to be the carboxy-terminus of the protein based on the presence of a stop codon (TAA) adjacent to it. The nucleic acid sequence preceding the couplet TC (positions 305-306) is presumed to be an intron (non-coding sequence) based on the presence of a consensus acceptor sequence (i.e. a pyrimidine-rich stretch, TTCTCCCTTTTCGTTCCT, followed by AG) and the presence of a stop rather than a basic residue in the appropriate position of the derived amino acid sequence.

bBMP-3 is therefore characterized by the DNA and amino acid sequence of Table IV A and Table IV B. The peptide sequence of this clone is 175 amino acids in length and is encoded by the DNA sequence from nucleotide #414 through nucleotide #746 of Table IV A and nucleotide #305 through nucleotide #493 of Table IV B.

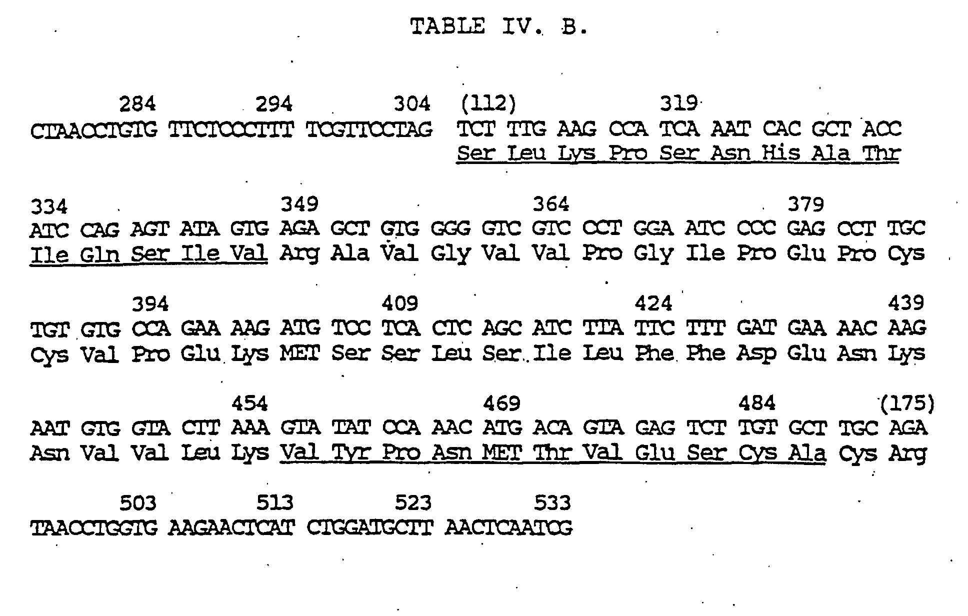

### EXAMPLE V

### Human BMP-3

The sequences of BMP-3 as shown in Tables IV A+B have significant homology to the beta (B) and beta (A) subunits of the inhibins. The inhibins are a family of hormones which are presently being investigated for use in contraception. See, A. J. Mason et al, Nature, 318:659-663 (1985). To a lesser extent they are also homologous to Mullerian inhibiting substance (MIS), a testicular glycoprotein that causes regression of the Mullerian duct during development of the male embryo and transforming growth factor-beta (TGF-b) which can inhibit or stimulate growth of cells or cause them to differentiate.

Because bovine and human bone growth factor genes are presumed to be significantly homologous, oligonucleotide probes which have been shown to hybridize to the bovine DNA sequence of Table IV.A and IV.B are used to screen a human genomic library. A human genomic library (Toole et al.; supra) is screened using these probes, and presumptive positives are isolated and DNA sequence obtained as described above. Evidence that this recombinant encodes a portion of the human bone inductive factor molecule relies on the bovine/human protein and gene structure homologies.

Once a recombinant bacteriophage containing DNA encoding a portion of the human BMP-3 molecule is obtained the human coding sequence is used as a probe as described in Example V (A) to identify a human cell line or tissue which synthesizes BMP-3. mRNA is selected by oligo (dT) cellulose chromatography and cDNA is synthesized and cloned in lambda gt10 by established techniques (Toole et al., supra).

Alternatively, the entire gene encoding this human bone inductive factor can be identified and obtained in additional recombinant clones if necessary. Additional recombinants containing further 3' or 5' regions of this human bone inductive factor gene can be obtained by identifying unique DNA sequences at the end(s) of the original clone and using these as probes to rescreen the human genomic library. The gene can then be reassembled in a single plasmid by standard molecular biology techniques and amplified in bacteria. The entire human BMP-3 factor gene can then be transferred to an appropriate expression vector. The expression vector containing the gene is then transfected into a mammalian cell, e.g. monkey COS cells, where the human gene is transcribed and the RNA correctly spliced. Media from the transfected cells are assayed for bone inductive factor activity as described herein as an indication that the gene is complete. mRNA is obtained from these cells and cDNA synthesized from this mRNA source and cloned. The procedures described above may similarly be employed to isolate other species' bone inductive factor of interest by utilizing the bovine bone inductive factor and/or human bone inductive factor as a probe source. Such other species' bone inductive factor may find similar utility in, inter alia, fracture repair.

### EXAMPLE VI

### Expression of Bone Inductive Factors.

In order to produce bovine, human or other mammalian bone inductive factors, the DNA encoding it is transferred into an appropriate expression vector and introduced into mammalian cells by conventional genetic engineering techniques.

One skilled in the art can construct mammalian expression vectors by employing the sequence of Tables IV A+B or other modified sequences and known vectors, such as pCD [Okayama et al., Mol. Cell Biol., 2:161-170 (1982)] and pJL3, pJL4 [Gough et al., EMBO J., 4:645-653 (1985)]. 'The transformation of these vectors into appropriate host cells can result in expression of osteoinductive factors. One skilled in the art could manipulate the sequences of Tables IV A+B by eliminating or replacing the mammalian regulatory sequences flanking the coding sequence with bacterial sequences to create bacterial vectors for intracellular or extracellular expression by bacterial cells. For example, the coding sequences could be further manipulated (e.g.. ligated to other known linkers or modified by deleting non-coding sequences there-from or altering nucleotides therein by other known techniques). The modified bone inductive factor coding sequence could then be inserted into a known bacterial vector using procedures such as described in T. Taniguchi et al., Proc. Natl Acad. Sci. USA, 77:5230-5233 (1980). This exemplary bacterial vector could then be transformed into bacterial host cells and bone inductive factor expressed thereby. For a strategy for producing extracellular expression of bone inductive factor in bacterial cells., see, e.g. European patent application EPA 177,343.

Similar manipulations can be performed for the construction of an insect vector [See, e.g. procedures described in published European patent application 155,476] for expression in insect cells. A yeast vector could also be constructed employing yeast regulatory sequences for intracellular or extracellular expression of the factors of the present invention by yeast cells. [See, e.g., procedures described in published PCT application WO86/00639 and European patent application EPA 123,289].

A method for producing high levels of an osteoinductive factor of the invention from mammalian cells involves the construction of cells containing multiple copies of the heterologous bone inductive factor gene. The heterologous gene can be linked to an amplifiable marker, e.g. the dihydrofolate reductase (DHFR) gene for which cells containing increased gene copies can be selected for propagation in increasing concentrations of methotrexate (MTX) according to the procedures of Kaufman and Sharp, J. Mol. Biol., 159:601-629 (1982). This approach can be employed with a number of different cell types.

For example, a plasmid containing a DNA sequence for a bone inductive factor of the invention in operative association with other plasmid sequences enabling expression thereof and the DHFR expression plasmid pAdA26SV(A)3 [Kaufman and Sharp, Mol. Cell. Biol., 2:1304 (1982)] can be co-introduced into DHFR-deficient CHO cells, DUKX-BII, by calcium phosphate coprecipitation and transfection. DHFR expressing transformants are selected for growth in alpha media with dialyzed fetal calf serum, and subsequently selected for amplification by growth in increasing concentrations of MTX (sequential steps in 0.02, 0.2, 1.0 and 5uM MTX) as described in Kaufman et al., Mol Cell Biol., 5:1750 (1983). Transformants are cloned, and biologically active bone inductive factor expression is monitored by rat bone formation assay. Bone inductive factor expression should increase with increasing levels of MTX resistance. Similar procedures can be followed to produce other bone inductive factors.

Alternatively, the human gene is expressed directly, as described above. Active bone inductive factor may be produced in bacteria or yeast cells. However the presently preferred expression system for biologically active recombinant human bone inductive factor is stably transformed CHO cells.

### Example VII

### Biological Activity of Expressed Bone Inductive Factor

To measure the biological activity of the expressed bone inductive factor obtained in Example VI above. The factor is partially purified on a Heparin Sepharose column. 4 ml of transfection supernatant from one 100 mm dish is concentrated approximately 10 fold by ultrafiltration on a YM 10 membrane and then dialyzed against 20mM Tris, 0.15 M NaCl, pH 7.4 (starting buffer). This material is then applied to a 1.1 ml Heparin Sepharose column in starting buffer. Unbound proteins are removed by an 8 ml wash of starting buffer, and bound proteins are desorbed by a 3-4 ml wash of 20 mM Tris, 2.0 M NaCl, pH 7.4.

The proteins bound by the Heparin column are concentrated approximately 10-fold on a Centricon 10 and the salt reduced by diafiltration with 0.1% trifluoroacetic acid. The appropriate amount of this solution is mixed with 20 mg of rat matrix and then assayed for in vivo bone and cartilage formation as previously described in Example III. A mock transfection supernatant fractionation is used as a control.

The implants containing rat matrix to which specific amounts of human BMP-3 have been added are removed from rats after seven days and processed for histological evaluation. Representative sections from each implant are stained for the presence of new bone mineral with von Kossa and acid fuschin, and for the presence of cartilage-specific matrix formation using toluidine blue. The types of cells present within the section, as well as the extent to which these cells display phenotype are evaluated.

The procedures described above may be employed to isolate other bone inductive factors of interest by utilizing the bovine bone inductive factors and/or human bone inductive factors as a probe source. Such other bone inductive factors may find similar utility in, inter alia, fracture repair.

## Claims (Claims for the following Contracting State(s): BE, CH, LI, DE, FR, GB, IT, LU, NL, SE)

1. A gene encoding bovine BMP-3 comprising the following DNA sequence:

383 393 403 413 428
GAGGAGGAAG CGGTCTACGG GGGTCCTTCT GCCTCTGCAG AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGCCATTG TTTTTTGTCC
Ala Tyr Tyr Cys Ser Gly Ala Cys Gln Phe Pro MET Pro Lys

776 786
TGTCCTTCCC ATTTCCATAG ; and

284 294 304 319
CTAACCTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

2. A gene encoding bovine BMP-3 having the amino acid sequence given in claim 1.

3. A gene encoding a protein exhibiting at least the property of BMP-3 to indues the formation of bone and comprising a DNA sequence:
(a) which differs from a DNA sequence of claim 1 in codon sequence due to the degeneracy of the genetic code;
(b) which hybridizes with a DNA sequence of claim 1 or section (a), above, under stringent hybridization conditions; or
(c) which represents a fragment, or allelic variation of a DNA sequence of claim. 1.

454                          469                          484
AAT GTG GTA CTT AAA GTA TAT CCA AAC ATG ACA GTA GAG TCT TGT GCT TGC AGA
Asn Val Val Leu Lys Val Tyr Pro Asn MET Thr Val Glu Ser Cys Ala Cys Arg

503        513        523        533
TAACCTGGTG AAGAACTCAT CTGGATGCTT AACTCAATCG.

4. The DNA sequence of claim 3, which encodes human BMP-3.

5. The DNA sequence of claim 3 or 4, which is a genomic DNA sequence.

6. The DNA sequence of claim 3 or 4, which is a cDNA sequence.

7. A vector containing the gene or DNA sequence of any one of claims 1 to 6 in operative association with an expression control sequence.

8. A cell transformed with a vector of claim 7.

9. The cell of claim 8 which is a mammalian cell, a bacterial cell, an insect cell or a yeast cell.

10. The cell of claim 9 which is a CHO cell.

11. A protein exhibiting properties of BMP-3 which is encoded by the gene or DNA sequence of any one of claims 1 to 6.

12. A protein exhibiting properties of BMP-3 which is produced by the steps of culturing in a suitable culture medium a cell transformed with an expression vector comprising a gene or a DNA sequence of any one of claims 1 to 6, and recovering said protein from said culture medium.

13. A process for producing the protein of claim 11 or 12, comprising the steps of culturing in a suitable culture medium the cell of claim 9 and isolating said protein from said culture medium.

14. A pharmaceutical composition comprising the protein of claim 11 or 12 and a pharmaceutically acceptable vehicle.

15. The pharmaceutical composition of Claim 14, further comprising a matrix capable of delivering the composition to the site of the bone or cartilage defect and providing a structure for inducing bone or cartilage formation.

16. The pharmaceutical composition of claim 15, wherein said matrix comprises hydroxyapatite, collagen, polylactic acid or tricalcium phosphate.

17. Use of a protein of claim 11 or 12 for the preparation of a pharmaceutical composition for inducing bone or cartilage formation.

## Claims (Claims for the following Contracting State(s): AT)

1. A method for the preparation of a gene encoding bovine BMP-3 comprising the following DNA sequence:

383 393 403 413 428
GAGGAGGAAG CGGTCTACGG GGGTCCTTCT GCCTCTGCAG AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 758 768
GCC TAT TAC TGC TCC GGA GCG TGC CAG TTC CCC ATG CCA AAG GTAGCCATTG TTTTTGTCC-
Ala Tyr Tyr Cys Ser Gly Ala Cys Gln Phe Pro MET Pro Lys

778 788
TGTCCTTCCC ATTTCCATAG ; and

284 294 304 319
CTAACCTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

2. The method of claim 1, wherein the gene encoding the BMP-3 has the amino acid sequence given in claim 1.

3. The method of claim 1, wherein a gene encoding a protein exhibiting at least the property of BMP-3 to induce the formation of bone comprises a DNA sequence:
(a) which differs from a DNA sequence obtained according to the method of claim 1 in codon sequence due to the degeneracy of the genetic code;
(b) which hybridises with a DNA sequence obtained according to the method of claim 1 or section (a), above under stringent hybridisation conditions; or
(c) which represents a fragment, or allelic variation of a DNA sequence obtained according to the method of claim 1. 
454 469 484
AAT GTG GTA CTT AAA GTA TAT CCA AAC ATG ACA GTA GAG TCT TGT GCT TGC AGA
Asn Val Val Leu Lys Val Tyr Pro Asn MET Thr Val Glu Ser Cys Ala Cys Arg

503 513 523 533
TAACCTGGTG AAGAACTCAT CTGGATGCTT AACTCAATCG.
 said method comprising screening of a recombinant bovine genomic library with probes consisting of pools of oligonucleotides designed on the basis of partial amino acid sequences and isolating a positive clone.

4. The method of claim 3, wherein the DNA sequence encodes human BMP-3.

5. The method of claim 3 or 4, wherein the DNA sequence is a genomic DNA sequence.

6. The method of claim 3 or 4, wherein the DNA sequence is a cDNA sequence.

7. A method for the preparation of a recombinant vector comprising inserting the gene or DNA sequence obtained according to a method of any one of claims 1 to 6 in operative association with an expression control sequence in a suitable vector.

8. A cell transformed with a vector prepared according to the method of claim 7.

9. The cell of claim 8 which is a mammalian cell, a bacterial cell, an insect cell or a yeast cell.

10. The cell of claim 9 which is a CHO cell.

11. A process for producing a protein exhibiting properties of BMP-3 comprising the steps of culturing in a suitable culture medium the cell of claim 9 and isolating said protein from said culture medium.

12. A method for the preparation of a pharmaceutical composition comprising combining the protein produced according to the method of claim 11 with a pharmaceutically acceptable vehicle.

13. The method of claim 12, wherein the pharmaceutical composition further comprises a matrix capable of delivering the composition to the site of the bone or cartilage defect and providing a structure for inducing bone or cartilage formation.

14. The method of claim 13, wherein said matrix of said pharmaceutical composition comprises hydroxyapatite, collagen, polylactic acid or tricalcium phosphate.

15. Use of a protein of claim 11 for the preparation of a pharmaceutical composition for inducing bone or cartilage formation.

16. A gene encoding bovine BMP-3 comprising the following DNA sequence:

383 393 403 413 428
GAGGAGGAAG CGGTCTACGG GGGTCCTTCT GCCTCTGCAG AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGCCATTG TTTTTGTCC-
Ala Tyr Tyr Cys Ser Gly Ala Cys Gln Phe Pro MET Pro Lys

776 786
TGTCCTTCCC ATTTCCATAG ; and

284 294 304 319
CTAACCTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

17. A gene encoding bovine BMP-3 having the amino acid sequence given in claim 16.

18. A gene encoding a protein exhibiting at least the property of BMP-3 to induce the formation of bone and comprising a DNA sequence:
(a) which differs from a DNA sequence of claim 16 in codon sequence due to the degeneracy of the genetic code;
(b) which hybridises with a DNA sequence of claim 16 or section (a), above, under stringent hybridisation conditions; or
(c) which represents a fragment, or allelic variation of a DNA sequence of claim 16.

454 469 484
AAT GTG GTA CTT AAA GTA TAT CCA AAC ATG ACA GTA GAG TCT TGT GCT TGC AGA
Asn Val Val Leu Lys Val Tyr Pro Asn Met Thr Val Glu Ser Cys Ala Cys Arg

503 513 523 533
TAACCTGGTG AAGAACTGAT CTGGATGCTT AACTCATCG.

19. The DNA sequence of claim 18, which encodes human BMP-3.

20. The DNA sequence of claim 18 or 19, which is a genomic DNA sequence.

21. The DNA sequence of claim 18 or 19, which is a cDNA sequence.

22. A vector containing the gene or DNA sequence of any one of claims 16 to 21 in operative association with an expression control sequence.

23. A cell transformed with a vector of claim 22.

24. The cell of claim 23 which is a mammalian cell, a bacterial cell, an insect cell or a yeast cell.

25. The cell of claim 24, which is a CHO cell.

26. A protein exhibiting properties of BMP-3 which is encoded by the gene or DNA sequence of any one of claims 1 to 21.

27. A protein exhibiting properties of BMP-3 which is produced by the steps of culturing in a suitable culture medium a cell transformed with an expression vector comprising a gene or a DNA sequence of any one of claims 1 to 21 and recovering said protein from said culture medium.

28. A pharmaceutical composition comprising the protein of claim 26 or 27 and a pharmaceutically acceptable vehicle.

29. The pharmaceutical composition of claim 29, further comprising a matrix capable of delivering the composition to the site of the bone or cartilage defect and providing a structure for inducing bone or cartilage formation.

30. The pharmaceutical composition of claim 29, wherein said matrix comprises hydroxyapatite, collagen, polylactic acid or tricalcium phosphate.

## Patentansprüche (Patentansprüche für folgende(n) Vertragsstaat(en): BE, CH, LI, DE, FR, GB, IT, LU, NL, SE)

1. Gen, das bovines BMP-3 codiert, welches die folgende DNA-Sequenz umfasst:

383 393 403 413 426
GAGGAGGAAG CGGTCTACGG GGGTCCTTCT GCCTCTGCAG AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGCCATTG TTTTTTGTCC-
Ala Tyr Tyr Cys Ser Gly Ala Cys Gln Phe Pro MET Pro Lys

776 786
TGTCCTTCCC ATTTCCATAG ; and

284 294 304 319
CTAACCTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

454 469 484
AAT GTG GTA CTT AAA GTA TAT CCA AAC ATG ACA GTA GAG TCT TGT GCT TGC AGA
Asn Val Val Leu Lys Val Tyr Pro Asn MET Thr Val Glu Ser Cys Ala Cys Arg

503 513 523 533
TAACCTGGTG AAGAACTGAT CTGGATGCTT AACTCATCG.

2. Gen, das bovines BMP-3 codiert, welches die Aminosäuresequenz aufweist, die in Anspruch 1 angegeben ist.

3. Gen, das ein Protein codiert, das mindestens die Eigenschaft von BMP-3 aufweist, die Bildung von Knochen zu induzieren, und eine DNA-Sequenz umfasst:
(a) die sich von einer DNA-Sequenz nach Anspruch 1 in der Codonsequenz auf Grund der Degeneriertheit des genetischen Codes unterscheidet;
(b) die mit einer DNA-Sequenz nach Anspruch 1 oder Absatz (a) oben unter stringenten Hybridisierungsbedingungen hybridisiert; oder
(c) die ein Fragment oder eine allelische Variation einer DNA-Sequenz nach Anspruch 1 darstellt.

4. DNA-Sequenz nach Anspruch 3, die menschliches BMP-3 codiert.

5. DNA-Sequenz nach Anspruch 3 oder 4, die eine genomische DNA-Sequenz ist.

6. DNA-Sequenz nach Anspruch 3 oder 4, die eine cDNA-Sequenz ist.

7. Vektor, der das Gen oder die DNA-Sequenz nach einem der Ansprüche 1 bis 6 in funktioneller Verknüpfung mit einer Expressionskontrollsequenz enthält.

8. Zelle, transformiert mit einem Vektor nach Anspruch 7.

9. Zelle nach Anspruch 8, die eine Säugerzelle, eine Bakterienzelle, eine Insektenzelle oder eine Hefezelle ist.

10. Zelle nach Anspruch 9, die eine CHO-Zelle ist.

11. Protein, das Eigenschaften von BMP-3 aufweist, das von dem Gen oder der DNA-Sequenz nach einem der Ansprüche 1 bis 6 codiert wird.

12. Protein, das Eigenschaften von BMP-3 aufweist, das durch die Schritte des Züchtens in einem geeigneten Kulturmedium von einer Zelle, die mit einem Expressionsvektor transformiert ist, der ein Gen oder eine DNA-Sequenz nach einem der Ansprüche 1 bis 6 umfasst, und des Gewinnens des Proteins aus dem Kulturmedium hergestellt wird.

13. Verfahren zur Herstellung des Proteins nach Anspruch 11 oder 12, umfassend die Schritte des Züchtens der Zelle nach Anspruch 9 in einem geeigneten Kulturmedium und des Isolierens des Proteins aus dem Kulturmedium.

14. Arzneimittel, umfassend das Protein nach Anspruch 11 oder 12 und ein pharmazeutisch verträgliches Vehikel.

15. Arzneimittel nach Anspruch 14, das zusätzlich eine Matrix umfasst, die das Mittel an die Stelle des Knochen- oder Knorpeldefekts bringen und eine Struktur zum Induzieren von Knochen- oder Knorpelbildung zur Verfügung stellen kann.

16. Arzneimittel nach Anspruch 15, wobei die Matrix Hydroxyapatit, Kollagen, Polymilchsäure oder Tricalciumphosphat umfasst.

17. Verwendung eines Proteins nach Anspruch 11 öder 12 für die Herstellung eines Arzneimittels zum Induzieren von Knochen- oder Knorpelbildung.

## Patentansprüche (Patentansprüche für folgende(n) Vertragsstaat(en): AT)

1. Verfahren zur Herstellung eines Gens, das bovines BMP-3 codiert, welches die folgende DNA-Sequenz umfasst: 
383 393 403 413 428
GAGGAGGAAG CGGTCTACGG GGGTCCTTCT GCCTCTGCAG · AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGCCATTG TTTTTTGTCC-
Ala Tyr Tyr Cys Ser Gly Ala Cys Gln Phe Pro MET Pro Lys

776 786
TGTCCTTCCC ATTTCCATAG ; and

284 294 304 319
CTAACCTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

454 469 484
AAT GTG GTA CTT AAA GTA TAT CCA AAC ATG ACA GTA GAG TCT TGT GCT TGC AGA
Asn Val Val Leu Lys Val Tyr Pro Asn MET Thr Val Glu Ser Cys Ala Cys Arg

503 513 523 533
TAACCTGGTG AAGAACTCAT CTGGATGCTT AACTCAATCG.
 wobei das Verfahren das Durchmustern einer rekombinanten bovinen genomischen Bibliothek mit Sonden, die aus Pools von Oligonucleotiden bestehen, die auf der Grundlage von partiellen Aminosäuresequenzen hergestellt wurden, und das Isolieren eines positiven Klons umfasst.

2. Verfahren nach Anspruch 1, wobei das Gen, das BMP-3 codiert, die Aminosäuresequenz aufweist, die in Anspruch 1 angegeben ist.

3. Verfahren nach Anspruch 1, wobei ein Gen, das ein Protein codiert, welches mindestens die Eigenschaft von BMP-3 zeigt, die Bildung von Knochen zu induzieren, eine DNA-Sequenz umfasst:
(a) die sich von einer DNA-Sequenz, die gemäß dem Verfahren nach Anspruch 1 erhalten wurde, in der Codonsequenz auf Grund der Degeneriertheit des genetischen Codes unterscheidet;
(b) die mit einer DNA-Sequenz, die gemäß dem Verfahren nach Anspruch 1 oder Absatz (a) oben erhalten wurde, unter stringenten Hybridisierungsbedingungen hybridisiert; oder
(c) die ein Fragment oder eine allelische Variation einer DNA-Sequenz darstellt, die gemäß dem Verfahren nach Anspruch 1 erhalten wurde.

4. Verfahren nach Anspruch 3, wobei die DNA-Sequenz menschliches BMP-3 codiert.

5. Verfahren nach Anspruch 3 oder 4, wobei die DNA-Sequenz eine genomische DNA-Sequenz ist.

6. Verfahren nach Anspruch 3 oder 4, wobei die DNA-Sequenz eine cDNA-Sequenz ist.

7. Verfahren zur Herstellung eines rekombinanten Vektors, umfassend das Einfügen des Gens oder der DNA-Sequenz, die gemäß einem Verfahren nach einem der Ansprüche 1 bis 6 erhalten wurde, in funktioneller Verknüpfung mit einer Expressionskontrollsequenz in einen geeigneten Vektor.

8. Zelle, transformiert mit einem Vektor, der gemäß dem Verfahren nach Anspruch 7 hergestellt wurde.

9. Zelle nach Anspruch 8, die eine Säugerzelle, eine Bakterienzelle, eine Insektenzelle oder eine Hefezelle ist.

10. Zelle nach Anspruch 9, die eine CHO-Zelle ist.

11. Verfahren zur Herstellung eines Proteins, das Eigenschaften von BMP-3 aufweist, umfassend die Schritte des Züchtens der Zelle nach Anspruch 9 in einem geeigneten Kulturmedium und des Isolierens des Proteins aus dem Kulturmedium.

12. Verfahren zur Herstellung eines Arzneimittels, umfassend das Kombinieren des Proteins, das gemäß dem Verfahren nach Anspruch 11 hergestellt wurde, mit einem pharmazeutisch verträglichen Vehikel.

13. Verfahren nach Anspruch 12, wobei das Arzneimittel zusätzlich eine Matrix umfasst, die das Mittel an die Stelle des Knochen- oder Knorpeldefekts bringen und eine Struktur für das Induzieren von Knochen- oder Knorpelbildung zur Verfügung stellen kann.

14. Verfahren nach Anspruch 13, wobei die Matrix des Arzneimittels Hydroxyapatit, Kollagen, Polymilchsäure oder Tricalciumphosphat umfasst.

15. Verwendung eines Proteins nach Anspruch 11 für die Herstellung eines Arzneimittels zum Induzieren von Knochen- oder Knorpelbildung.

16. Gen, das bovines BMP-3 codiert, welches die folgende DNA-Sequenz umfasst:

383 393 403 413 428
GAGGAGGAAG CGGTCTACGG GGGTCCTTCT GCCTCTGCAG AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGCCATTG TTTTTTGTCC-
Ala Tyr Tyr Cys Ser Gly Ala Cys Gln Phe Pro MET Pro Lys

776 786
TGTCCTTCCC ATTTCCATAG ; and

284 294 304 319
CTAACTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

454 469 484
AAT GTG GTA CTT AAA GTA TAT CCA AAC ATG ACA GTA GAG TCT TGT GCT TGC AGA
Asn Val Val Leu Lys Val Tyr Pro Asn MET Thr Val Glu Ser Cys Ala Cys Arg

503 513 523 533
TAACCTGGTG AAGAACTCAT CTGGATGCTT AACTCATCG.

17. Gen, das bovines BMP-3 codiert, welches die Aminosäuresequenz aufweist, die in Anspruch 16 angegeben ist.

18. Gen, das ein Protein codiert, das mindestens die Eigenschaft von BMP-3 aufweist, die Bildung von Knochen zu induzieren, und eine DNA-Sequenz umfasst:
(a) die sich von einer DNA-Sequenz nach Anspruch 16 in der Codonsequenz auf Grund der Degeneriertheit des genetischen Codes unterscheidet;
(b) die mit einer DNA-Sequenz nach Anspruch 16 oder Absatz (a) oben unter stringenten Hybridisierungsbedingungen hybridisiert; oder
(c) die ein Fragment oder eine allelische Variation einer DNA-Sequenz nach Anspruch 16 darstellt.

19. DNA-Sequenz nach Anspruch 18, die menschliches BMP-3 codiert.

20. DNA-Sequenz nach Anspruch 18 oder 19, die eine genomische DNA-Sequenz ist.

21. DNA-Sequenz nach Anspruch 18 oder 19, die eine cDNA-Sequenz ist.

22. Vektor, der das Gen oder die DNA-Sequenz nach einem der Ansprüche 16 bis 21 in funktioneller Verknüpfung mit einer Expressionskontrollsequenz enthält.

23. Zelle, transformiert mit einem Vektor nach Anspruch 22.

24. Zelle nach Anspruch 23, die eine Säugerzelle, eine Bakterienzelle, eine Insektenzelle oder eine Hefezelle ist.

25. Zelle nach Anspruch 24, die eine CHO-Zelle ist.

26. Protein, das Eigenschaften von BMP-3 aufweist, das von dem Gen oder der DNA-Sequenz nach einem der Ansprüche 1 bis 21 codiert wird.

27. Protein, das Eigenschaften von BMP-3 aufweist, das durch die Schritte des Züchtens in einem geeigneten Kulturmedium von einer Zelle, die mit einem Expressionsvektor transformiert ist, der ein Gen oder eine DNA-Sequenz nach einem der Ansprüche 1 bis 21 umfasst, und des Gewinnens des Proteins aus dem Kulturmedium hergestellt wird.

28. Arzneimittel, das das Protein nach Anspruch 26 oder 27 und ein pharmazeutisch verträgliches Vehikel umfasst.

29. Arzneimittel nach Anspruch 29, das zusätzlich eine Matrix umfasst, die das Mittel an die Stelle des Knochen- oder Knorpeldefekts bringen und eine Struktur zum Induzieren von Knochen- oder Knorpelbildung zur Verfügung stellen kann.

30. Arzneimittel nach Anspruch 29, wobei die Matrix Hydroxyapatit, Kollagen, Polymilchsäure oder Tricalciumphosphat umfasst.

## Revendications (Revendications pour l'(les) Etat(s) contractant(s) suivant(s): BE, CH, LI, DE, FR, GB, IT, LU, NL, SE)

1. Gène codant pour la BMP-3 bovine comprenant la séquence d'ADN suivante :

383 393 403 413 428
GAGGAGGAAG CAGTCTACGG GGGTCCTTCT GCCTCTGCAG AAC AAT GAG CTT CCT GGC GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGCCATTG TTTTTTGTCC
Ala Tyr Tyr Cys Ser Gly Ala Cys Gln Phe Pro MET Pro Lys

776 786
TGTCCTTCCC ATTTCCATAG ; et

284 294 304 319
CTAACTTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

454 469 484
AAT GTG GTA CTT AAA GTA TAT CCA AAC ATG ACA GTA GAG TCT TGT GCT TGC AGA
Asn Val Val Leu Lys Val Tyr Pro Asn Met Thr Val Glu Ser Cys Ala Cys Arg

503 513 523 533
TAACCTGTTG AAGAACTGAT CTGGATGCTT AACTGATCC.

2. Gêne codant pour la BMP-3 bovine ayant la séquence d'acides aminés donnée à la revendication 1.

3. Gène codant pour une protéine montrant au moins les propriétés de la BMP-3 pour induire la formation osseuse et comprenant une séquence d'ADN :
(a) qui diffère d'une séquence d'ADN de la revendication 1 dans la séquence de codons du fait de la dégénérescence du code génétique ;
(b) qui s'hybride avec une séquence d'ADN de la revendication 1 ou du paragraphe (a) ci-dessus dans des conditions d'hybridation stringentes; ou
(c) représente un fragment ou une variation allélique d'une séquence d'ADN de la revendication 1.

4. Séquence d'ADN suivant la revendication 3, qui code pour la BMP-3 humaine.

5. Séquence d'ADN suivant la revendication 3 ou 4, qui est une séquence d'ADN génomique.

6. Séquence d'ADN suivant la revendication 3 ou 4, qui est une séquence d'ADNc.

7. Vecteur contenant le gène ou la séquence d'ADN suivant l'une quelconque des revendications 1 à 6, en association opérationnelle avec une séquence de contrôle d'expression.

8. Cellule transformée avec un vecteur de la revendication 7.

9. Cellule suivant la revendication 8, qui est une cellule mammifère, une cellule bactérienne, une cellule d'insecte ou une cellule de levure.

10. Cellule suivant la revendication 9, qui est une cellule CHO.

11. Protéine montrant des propriétés de la BMP-3, qui est codée par le gène ou la séquence d'ADN de l'une quelconque des revendications 1 à 6.

12. Protéine montrant des propriétés de la BMP-3, qui est produite par les étapes de culture dans un milieu de culture approprié d'une cellule transformée avec un vecteur d'expression comprenant un gène ou une séquence d'ADN de l'une quelconque des revendications 1 à 6, et de récupération de ladite protéine du milieu de culture précité.

13. Procédé de production de la protéine suivant l'une ou l'autre des revendications 11 ou 12, comprenant les étapes de culture dans un milieu de culture approprié de la cellule de la revendication 9 et d'isolement de ladite protéine du milieu de culture précité.

14. Composition pharmaceutique comprenant la protéine suivant l'une ou l'autre des revendications 11 ou 12 et un véhicule pharmaceutiquement acceptable.

15. Composition pharmaceutique suivant la revendication 14, comprenant en outre une matrice pouvant distribuer la composition au site de l'anomalie osseuse ou cartilagineuse et fournir une structure pour induire une formation osseuse ou cartilagineuse.

16. Composition pharmaceutique suivant la revendication 15, dans laquelle ladite matrice comprend de l'hydroxyapatite, du collagène, de l'acide polylactique ou du phosphate tricalcique.

17. Utilisation d'une protéine suivant l'une ou l'autre des revendications 11 ou 12 pour la préparation d'une composition pharmaceutique pour induire une formation osseuse ou cartilagineuse.

## Revendications (Revendications pour l'(les) Etat(s) contractant(s) suivant(s): AT)

1. Procédé de préparation d'un gène codant pour le BMP-3 bovin comprenant la séquence d'ADN suivante : 
383 393 403 413 428
GAGCAGGAAG CGGTCGACGG GGGTCCTTCT GCCTCTGCAG · AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGCCATTG TTTTTGTCC-
Ala Tyr Tyr Cys Ser Gly Ala Cys Gln Phe Pro MET Pro Lys

776 786
TGTCCTTCCC ATTTCCATAG ; et

284 294 304 319
CTAACCTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

454 469 484
AAT GTG GTA CTT AAA GTA TAT CCA AAC ATG ACA GTA GAG TCT TGT GCT TGC AGA
Asn Val Val Leu Lys Val Tyr Pro Asn MET Thr Val Glu Ser Cys Ala Cys Arg

503 513 523 533
TAACTGGGTG AAGAACTCAT CTGGATGCTT AACTCATCC.
 ledit procédé comprenant le criblage d'une banque génomique recombinante de bovin avec des sondes comprenant des pools d'oligonucléotides conçues sur la base des séquences d'acides aminés partielles et l'isolement d'un clone positif.

2. Procédé selon la revendication 1, dans lequel le gène codant pour le BMP-3 a la séquence d'acides aminés donnée dans la revendication 1.

3. Procédé selon la revendicacion 1, dans lequel un gène codant pour une protéine présentant au mois la propriété du BMP-3 d'induction de la formation de l'os comprend une séquence d'ADN :
a) qui diffère d'une séquence d'ADN obtenue selon la procédé de la revendication 1 par la séquence de codons du fait de la dégénérescence du code génétique ;
b) qui s'hybride avec une séquence d'ADN obtenue selon le procédé de la revendication 1 ou la section a) ci-dessus dans des conditions d'hybridation stringentes ; ou
c) représente un fragment ou une variation allélique d'une séquence d'ADN obtenue selon le procédé de la revendication 1,

4. Procédé selon la revendication 3, dans lequel la séquence d'ADN code pour le BMP-3 humain.

5. Procédé selon la revendication 3 ou 4, dans lequel la séquence d'ADN est une séquence d'ADN génomique.

6. Procédé selon la revendication 3 ou 4, dans lequel la séquence d'ADN est une séquence d'ADNc.

7. Procédé de préparation d'un vecteur recombinant comprenant l'insertion du gène ou de la séquence d'ADN obtenue selon un procédé de l'une quelconque des revendications 1 à 6 en association opératoire avec une séquence de contrôle d'expression dans un vecteur adapté.

8. Cellule transformés avec un vecteur préparé selon le procédé de la revendication 7.

9. Cellule selon la revendication 8, qui est une cellule mammifère, une cellule bactérienne, une cellule d'insecte ou une cellule de levure.

10. Cellule selon la revendication 9, qui est une cellule CHO.

11. Procédé de production d'une protéine présentant les propriétés du BMP-3, comprenant les étapes de culture dans un milieu de culture adapté de la cellule de la revendication 9 et d'isolement de ladite protéine dudit milieu de culture.

12. Procédé de préparation d'une composition pharmaceutique comprenant la combinaison de la protéine produite selon le procédé de la revendication 11 avec un véhicule pharmaceutiquement acceptable.

13. Procédé selon la revendication 12, dans lequel la composition pharmaceutique comprend en outre une matrice capable de délivrer la composition sur le site du défaut de l'os ou du cartilage et la fourniture d'une structure pour induire la formation d'os ou de cartilage.

14. Procédé selon la revendication 13, dans lequel ladite matrice de ladite composition pharmaceutique comprend de l'hydroxyapatite, du collagène, de l'acide polylactique ou du phosphate tricalcique.

15. Utilisation d'une protéine selon la revendication 11 pour la préparation d'une composition pharmaceutique pour induire la formation d'os ou de cartilage.

16. Gène codant pour le BMP-3 bovin comprenant la séquence d'ADN suivante :

383 393 403 413 428
GAGGAGGAAG CAGTCTACGG GGGTCCTTCT GCCTCTGCAG AAC AAT GAG CTT CCT GGG GCA
Asn Asn Glu Leu Pro Gly Ala

443 458 473 488
GAA TAT CAG TAC AAG GAG GAT GAA GTA TGG GAG GAG AGG AAG CCT TAC AAG ACT
Glu Tyr Gln Tyr Lys Glu Asp Glu Val Trp Glu Glu Arg Lys Pro Tyr Lys Thr

503 518 533
CTT CAG ACT CAG CCC CCT GAT AAG AGT AAG AAC AAA AAG AAA CAG AGG AAG GGA
Leu Gln Thr Gln Pro Pro Asp Lys Ser Lys Asn Lys Lys Lys Gln Arg Lys Gly

548 563 578 593
CCT CAG CAG AAG AGT CAG ACG CTC CAG TTT GAT GAA CAG ACC CTG AAG AAG GCA
Pro Gln Gln Lys Ser Gln Thr Leu Gln Phe Asp Glu Gln Thr Leu Lys Lys Ala

608 623 638
AGA AGA AAG CAA TGG ATT GAA CCC CGG AAT TGT GCC AGA CGG TAC CTT AAA GTG
Arg Arg Lys Gln Trp Ile Glu Pro Arg Asn Cys Ala Arg Arg Tyr Leu Lys Val

653 668 683 698
GAC TTC GCA GAT ATT GGC TGG AGC GAA TGG ATT ATT TCC CCC AAG TCC TTC GAT
Asp Phe Ala Asp Ile Gly Trp Ser Glu Trp Ile Ile Ser Pro Lys Ser Phe Asp

713 728 743 756 766
GCC TAT TAC TGC TCC GGA GCG TGC CAG TTC CCC ATG CCA AAG GTAGGCCATG TTTTTGTCCG-
Ala Tyr Tyr Cys Ser Gly Ala Cys Gln Phe Pro MET Pro Lys

776 786
TGTCCTTCCC ATTTCCATAG ; et

284 294 304 319
CTAACCTGTG TTCTCCCTTT TCGTTCCTAG TCT TTG AAG CCA TCA AAT CAC GCT ACC
Ser Leu Lys Pro Ser Asn His Ala Thr

334 349 364 379
ATC CAG AGT ATA GTG AGA GCT GTG GGG GTC GTC CCT GGA ATC CCC GAG CCT TGC
Ile Gln Ser Ile Val Arg Ala Val Gly Val Val Pro Gly Ile Pro Glu Pro Cys

394 409 424 439
TGT GTG CCA GAA AAG ATG TCC TCA CTC AGC ATC TTA TTC TTT GAT GAA AAC AAG
Cys Val Pro Glu Lys MET Ser Ser Leu Ser Ile Leu Phe Phe Asp Glu Asn Lys

454 469 484
AAT GTG GTA CTT AAA GTA TAT CCA AAC ATG ACA GTA GAG TCT TGT GCT TGC AGA
Asn Val Val Leu Lys Val Tyr Pro Asn MET Thr Val Glu Ser Cys Ala Cys Arg

503 513 523 533
TAACCTGGTG AAGAACTCAT CTGGATGCTT AACTCATCC.

17. Gène codant pour le BMP-3 bovin ayant la séquence d'acides aminés donnée dans la revendication 16.

18. Gène codant pour une protéine présentant au moins la propriété du BMP-3 d'induction de la formation de l'es et comprenant une séquence d'ADN :
a) qui diffère d'une séquence d'ADN de la revendication 16 par la séquence de codons du fait de la dégénérescence du code génétique ;
b) qui s'hybride avec une séquence d'ADN de la revendication 16 ou de la section a) ci-dessus dans des conditions d'hybridation stringentes ou
c) représente un fragment ou une variation allélique d'une séquence d'ADN selon la revendication 16.

19. Séquence d'ADN selon la revendication 18, qui code pour le BMP-3 humain.

20. Séquence d'ADN selon la revendication 18 ou 19, qui est une séquence d'ADN génomique.

21. Séquence d'ADN selon la revendication 18 ou 19, qui est une séquence d'ADNc.

22. Vecteur contenant le gène ou la séquence d'ADN selon l'une quelconque des revendications 16 à 21 en association opératoire avec une séquence de contrôle d'expression.

23. Cellule transformée avec un vecteur selon la revendication 22.

24. Cellule selon la revendication 23, qui est une cellule mammifère, une cellule bactérienne, une cellule d'insecte ou une cellule de levure,

25. Cellule selon la revendication 24, qui est une cellule CHC.

26. Protéine présentant les propriétés du BMP-3 qui est encodée par le gène ou la séquence d'ADN selon l'une quelconque des revendications 1 à 21.

27. protéine présentant les propriétés du BMP-3 qui est produite par les étapes de culture dans un milieu de culture adapté d'une cellule transformée avec un vecteur d'expression comprenant un gêne ou la séquence d'ADN selon l'une quelconque des revendications 1 à 21, et de récupération de ladite protéine dudit milieu de culture.

28. Composition pharmaceutique comprenant la protéine selon la revendication 26 ou 27 et un véhicule pharmaceutiquement acceptable.

29. Composition pharmaceutique selon la revendication 29, comprenant en outre une matrice capable de délivrer la composition sur le site du défaut de l'os ou du cartilage et la fourniture d'une structure pour induire la formation d'os ou de cartilage.

30. Composition pharmaceutique selon la revendication 29, dans laquelle ladite matrice comprend de l'hydroxyapatite, du collagène, de l'acide polylactique ou du phosphate tricalcique.
